# EUROPEAN PATENT SPECIFICATION

(11) **EP 0 628 319 B1**
(45) Date of publication and mention of the grant of the patent: **05.09.2001**
(21) Application number: 94108667.0
(22) Date of filing: 07.06.1994
(51) Int. Cl.: A61L 15/07, B65D 75/68, B32B 27/06

(54) **Orthopedic casting material and hermetic package**
Material für orthopädische Stützverbände und hermetische Verpackung
Matière pour plâtrage orthopédique et emballage hermétique

(30) Priority: 11.06.1993 US 75815
(43) Date of publication of application: 14.12.1994
(73) Proprietor: MINNESOTA MINING AND MANUFACTURING COMPANY, St. Paul, Minnesota 55133-3427 (US)
(72) Inventor: Delmore, Michael D., c/o Minnesota Mining and, Saint Paul, Minnesota 55133-3427 (US); Yasis, Rafael M., c/o Minnesota Mining and, Saint Paul, Minnesota 55133-3427 (US)
(74) Representative: von Kreisler, Alek, Dipl.-Chem.

(56) References cited:
- EP-A- 0 471 220
- US-A- 4 139 643
- US-A- 4 598 826
- US-A- 4 609 578

## Description

### Field of the Invention

The present invention relates to the field of orthopedic splinting/casting materials and packaging for the same. More particularly, the present invention relates to curable orthopedic splinting/casting material in a heat sealed hermetic package which includes means for facilitating tearing of the package in a predetermined direction.

### Background of the Invention

Hermetic packaging constructed from heat sealable laminated materials is well-known. Many products are placed in such packages to extend their shelf life by sealing the products from exposure to air, water vapor, etc. Heat sealed hermetic packaging constructed from laminated sheet materials is especially popular because of its relatively low cost as compared to effectiveness.

The hermeticity of such packages is especially important in the field of packaging for curable orthopedic splinting/casting materials. Such products are discussed in U.S. Patent Nos. 4,433,680; 4,609,578; 4,667,661; 4,774,937; and 5,027,803.

Briefly, however, such products are extremely sensitive to ambient levels of water vapor and will harden (i.e., "cure") upon exposure to very low levels of water vapor. Once hardened, the product can no longer be used for its intended purpose and must be discarded. Because of their sensitivity, such products are typically packaged in single-use hermetic packages as constructed from composite laminate sheet materials.

Hermetically sealed packages constructed from laminated sheet materials are, however, typically difficult to open due to the nature of the materials used to manufacture them. The laminated sheet materials typically comprise three or more layers chosen for their strength, moisture impermeability, and/or heat sealability. The composite material is difficult to tear and is particularly difficulty to tear in a desired direction. As a result, users must typically first tear the package once and additionally tear, cut or, for example, open one of the heat sealed ends to provide a large enough opening to allow adequate access to the product inside.

One attempt to provide heat sealed hermetic packages which provide predictable tearing characteristics is discussed in U.S. Patent No. 4,598,826. Tear strips are laminated to the interior of the package to facilitate tearing of the package in a desired direction lying along the tear strips. Such packages are, however, difficult to manufacture as the tear strips must typically be introduced as the package is formed from the laminated sheet material. In addition, the hermeticity of the packages can be compromised if the tear strips are included in the heat seals because of their relatively large profile which is difficult to accommodate in the heat seals without forming leaks.

Another attempt at addressing the difficulties in opening such packages includes forming heat sealed packages with lines of weakness across one of the heat seals as described in U.S. Patent No. 4,139,643. The lines of weakness are provided to enhance the predictability of opening the packages. One disadvantage of this approach is that the package can only be predictably opened along the heat seals. In many instances that opening is not large enough to provide sufficient access to the product without further tearing or cutting to enlarge the opening in the package.

Another disadvantage of providing lines of weakness is the opportunity that they present for leaks into the package. Such features may be particularly vulnerable to flex cracking which can break the thin foil vapor barrier layer used in the laminate sheet materials.

Yet another approach to providing predictable opening characteristics for heat sealed hermetic packages is described in U.S. Patent No. 4,279,344. The packages discussed in that reference include a heat sealable inner layer, metal foil vapor barrier layer and outer layer to protect the metal foil. The heat sealable inner layers form a peelable bond which is easily separated by the user. One disadvantage to this approach is that the bond formed is designed to be weak enough to allow for easy separation which can lead to failure of the package to maintain its hermeticity during shipping and handling.

European Patent Publication No. EP 0 471 220 shows a packing pouch. The pouch includes a resin layer formed by melt extrusion and two parallel strings so that the pouch can be torn along one of the two strings when a tear is made between the two strings.

### Summary of the Invention

Generally, a kit of orthopedic casting/splinting materials comprises a sheet of curable casting/splinting material, and a substantially hermetic package containing the sheet of curable casting/splinting material. The package comprises laminate sheet material having an outer layer and a heat sealable inner layer bonded to the outer layer, and a heat seal bonding at least a portion of the inner layer of the laminate sheet material to form the package. A plurality of generally parallel fibers are substantially evenly spaced apart and disposed between the inner layer and the outer layer of the laminate sheet material to guide tearing the package along a predetermined direction defined by the fibers. The fibers are substantially evenly spaced along the laminate sheet material across substantially the entire laminate sheet material.

Thus, the invention as defined by claim 1 relates to a kit of orthopedic casting/splinting materials comprising:
a) a sheet (11) of curable casting/splinting material; and
b) a substantially hermetic package (10) containing the sheet (11) of curable casting/splinting material, the package (10) comprising:
   1) laminate sheet material (20; 30) having opposite side edges, and an outer layer (21, 22; 31, 32) and a heat sealable inner layer (24; 34) bonded to the outer layer (21, 22; 31, 32); and
   2) a heat seal (12, 14 and 16) bonding at least a portion of the inner layer (24; 34) of the laminate sheet material (20; 30) to form the package (10), the heat seal (12, 14 and 16) bonding the laminate sheet material (20; 30) to form opposite ends of the package (10), the ends of the package (10) defining a first direction extending between the ends and a second direction extending generally perpendicularly with respect to the first direction;
      the kit being characterized in that a plurality of generally parallel fibers (25; 35) are substantially evenly spaced apart and disposed within the inner layer (34) or between the inner layer (24) and the outer layer (21, 22; 31, 32), the fibers extending substantially along the first direction to guide tearing the package (10) along the first direction, the fibers (25; 35) being substantially evenly spaced apart along the laminate sheet material (20; 30) across substantially the entire laminate sheet material (20; 30) in the second direction such that the distance between either side of the laminate sheet material (20; 30) and at least one fiber (25; 35) is no greater than the distance the fibers (25; 35) are spaced apart.

The fiber of the tearing means provides a path along which the laminate sheet material of the package predictably tears when opened. The path extends along a direction which is sufficiently long to provide easy access to the product in the package.

The ability to provide predictable opening properties is especially useful in packaging for orthopedic splinting/casting materials because such packages are typically opened at the point of use to limit the product's exposure to moisture. In addition, easy access to the product in the package further limits the time needed to remove the product and, therefore, the product's exposure to moisture.

Furthermore, the ability to provide such properties without compromising the hermeticity of the package is particularly advantageous.

The preferred laminate sheet material comprises an inner heat sealable layer laminated to a metal foil layer which provides the desirable moisture-proof properties of the laminate. The outer surface of the metal layer is bonded to an outer layer which preferably provides toughness and puncture resistance to protect the vapor barrier properties of the metal foil.

In one aspect: of the invention, the fibers are laminated between the metal foil and outer layer. In that embodiment, the impact of the fibers on the heat seals is minimized because the fibers are farther from the heat sealed inner layers.

In another aspect of the invention, the fibers are laminated between the metal foil and the inner layer. In that embodiment, the fibers are better able to tear the metal foil layer, leading to more predictable tearing characteristics.

### Brief Description of the Drawings

FIGURE 1 is a perspective view, partially cut-away, of one embodiment of a package containing curable casting/splinting material according to the present invention.

FIGURE 2 is an enlarged partial cross-sectional view of one embodiment of a laminate for use in packages constructed according to the present invention.

FIGURE 3 is a an alternate embodiment of a laminate for use in packages constructed according to the present invention.

FIGURE 4 is a schematic diagram of one process of manufacturing one embodiment of a laminate for use in packages according to the present invention.

### Detailed Description of the Preferred and Alternate Embodiments

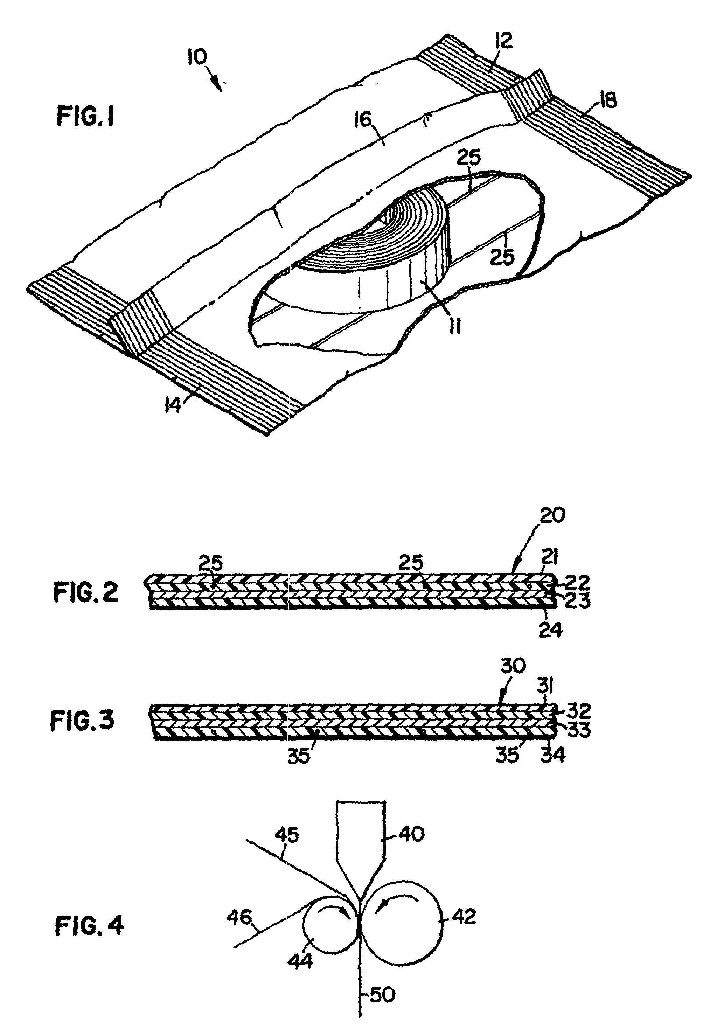

Fig. 1 depicts one embodiment of a pouch constructed of a laminate sheet material and containing a roll of curable casting/splinting material according to the present invention. As shown there, the pouch 10 includes end seals 12 and 14 and a fin seal 16 running lengthwise along the pouch 10. The pouch 10 also preferably includes a notch 18 to facilitate opening of the pouch 10. The pouch 10 is formed of laminate sheet material 20 which is discussed more fully below.

Located within the pouch is a roll of curable casting/splinting material 11. The preferred material 11 is moisture-curable and, most preferably, includes an isocyanate-functional resin which cures when exposed to moisture. Furthermore, although a roll is depicted in Fig. 1, it will be understood that the material 11 could be provided as a plurality of rolls or one or more flat sheets of material. Suitable curable casting/splinting materials are described in, for example, U.S. Patent Nos. 4,433,680; 4,609,578; 4,667,661; 4,774,937 and 5,027,803.

Those disclosures should not, however, be construed as limiting the scope of curable materials for packaging according to the present invention.

Although in the preferred embodiment, pouch 10 comprises two end seals 12 and 14 and a fin seal 16 (commonly referred to as a form, fill and seal pouch), it will be understood that hermetic heat sealed packages designed to incorporate laminate sheet material according to the present invention could take many forms and that the form described with respect to the preferred embodiment is only one version of the same. Alternate types of packages could include, but are not limited to, flat, two-dimensional pouches or three-dimensional, stand-up gussetted pouches.

Fig. 2 depicts an enlarged cross-sectional view of a portion of one embodiment of laminate sheet material used in a preferred embodiment of a package according to the present invention. The laminate 20 is a composite structure comprising layers 21, 22, 23 and 24. In the embodiment shown, layer 23 provides a vapor barrier through which air and water vapor transmission is severely limited.

Layers 21 and 22 preferably comprise a protective layer over the vapor barrier layer 23. Examples of potential materials for layers 21 and/or 22 include puncture resistant paper or plastic resin material. It is preferred that one of the outer protective layers is comprised of at least one layer of polyester, nylon or polypropylene which protects the preferred aluminum foil vapor barrier layer 23 from punctures and tears during shipping and handling.

In one preferred embodiment, outer layer 21 comprises biaxially oriented polypropylene (BOPP) having a thickness of 0.001 inch (0.025 millimeter). A layer 22 of ethylene acetate acrylic (EAA) having a thickness of 0.001 inch (0.025 millimeter) is bonded to one side of the layer 21 of BOPP to enhance the bonding of the BOPP outer layer 21 to the vapor barrier layer 23.

The EAA layer 22 is bonded to the vapor barrier layer 23 of aluminum foil (in the preferred embodiment) with a thickness of 0.00035 inch (0.00889 millimeter).

Although the preferred vapor barrier layer 23 is aluminum foil which is entirely free of even minute holes, it is known that such foils remain highly impervious to air and water vapor transmission even when they include a few widely scattered minute openings. In other words, by highly impervious it is meant that as little air and water vapor as possible can pass through the minute openings found in currently available metal foils used in laminate sheet material for packaging.

Furthermore, it will be understood that other materials can be substituted for the preferred aluminum foil provided they supply the necessary vapor barrier without significantly affecting the flexibility or heat/sealability of the laminate 20. Examples of alternate materials include, but are not limited to, metallized films, poly-vinylidene chloride coated films (e.g., SARAN coating), and ACLAR high moisture vapor permeable films (available from Allied Signal Inc, Morristown, New Jersey).

Bonded to the inside surface of vapor barrier layer 23 is an inner layer 24 of heat sealable material comprising a plastic resin film such as polypropylene, polyethylene or a polyethylene-polypropylene copolymer that is extruded and/or calendared directly onto layer 23. Most preferably, the heat sealable inner layer 24 is SURLYN brand resin (#1702) available from E.I. DuPont deNemours & Company, Wilmington, Delaware. It will, however, be understood that many other heat sealable materials could be substituted for the preferred variety. Examples of alternate materials include, but are not limited to, other polyethylenes, EAA or any low melting point thermoplastic material.

Inner layer 24 also preferably provides a barrier to prevent chemical reactions between the product in the packages and the vapor barrier layer 23. It also provides puncture and abrasion resistance to protect layer 23 from the product contained in any package formed from the laminate sheet material 20.

In the embodiment illustrated in Fig. 2, fibers 25 are contained within layer 22. The fibers are preferably spaced on one inch (25 mm) centers across the width of the packaging material. The preferred fiber 25 is a multi-fiber glass filament, although a monofilament fiber could be substituted. Multiple fiber filaments are generally preferred because their tensile strength is typically greater (for a given outside diameter) than the tensile strength of a monofilament fiber. The preferred fiber has a nominal diameter of 0.00033 inch (0.00838 millimeter/99 denier). It is constructed of glass fibers and has a tensile strength of 1.7 lbs. (0.77 kilogram). The preferred fibers are available from Owens Corning Fiberglas Company, Toledo, Ohio, under the designation ECD 450 1/0, although other similar fibers, including monofilament fibers, may be substituted provided their tensile strength to outside diameter ratio is sufficient for tearing as described below. In addition to multi- or mono-filament fibers, it will also be understood that the fibers can be of many different materials other than glass. Examples of alternate fiber materials include, but are not limited to, nylon, polyester, graphite and, aramid, such as available under the trademarks "KEVLAR" or "NOMEX" from E.I. DuPont deNemours & Company, Wilmington, Delaware.

The provision of a fiber 25 which has a relatively small diameter when compared to its tensile strength is important to operation of the present invention. The fibers 25, when inlaid into a laminate across a web preferably produce a laminate having a very flat cross-web profile. As a result, the roll is free of any visible signs of gauge band when the finished laminate is wound onto a roll. Gauge band is a phenomena which occurs with extruded laminates when the extrusion materials are not evenly distributed across the web. Uneven distribution of the cross web material can cause difficulties when the laminate is wound into finished roles which can telescope or otherwise be unstable. In addition, the fibers 25 are substantially evenly spaced apart across the laminate material 20 which also facilitates even winding of the laminate into finished rolls.

Furthermore, the size of the fibers 25 is preferably also small to minimize the effect of the fibers 25 in the heat seals 12 and 14 located at the ends of the preferred embodiment of the pouch 10 depicted in Fig. 1. Fibers 25 having large diameters could provide the opportunity for leakage at any heat seals in packages constructed with laminate sheet material according to the present invention.

When opened, the pouch 10 preferably tears along one or more of the fibers 25 located within the laminate sheet material 20 forming the pouch 10. It is contemplated that one or more of the fibers 25 may break during the tearing process. In that situation, the tear line will typically migrate to an adjacent fiber 25 and continue tearing along the length of the package 10. Whether the tear in the package follows the initial fiber 25 or whether the tear migrates to another fiber 25, the pouch 10 will typically be opened substantially along its full length, thereby allowing easy access to any product contained therein.

An alternate embodiment of a laminate sheet material 30 according to the present invention is depicted in Fig. 3. The laminate sheet material 30 comprises outer layers 31 and 32 bonded to a vapor barrier layer 33. As with laminate sheet material 20 in Fig. 2, outer layers 31 and 32 preferably combine to form a protective, puncture-resistant layer over the vapor barrier layer 33 to prevent punctures and tears of that layer 33 during handling.

On the opposite side of layer 33 is a heat sealable layer, preferably of SURLYN brand resin available from E.I. DuPont deNemours & Company, Wilmington, Delaware, which is used to heat seal packages formed using laminate sheet material 30.

In this embodiment, the heat seal layer 34 preferably incorporates fibers 35 as opposed to locating the fibers in the outer layers as described with respect to laminate sheet material 20 in Fig. 2 above. In all other aspects, the laminate sheet material 30 of Figure 3 is similar to the laminate sheet material 20 of Figure 2.

In each of the embodiments of laminate sheet materials 20 & 30 described above, the preferred materials used to manufacture the products consisted of the following: the biaxially oriented polypropylene film (BOPP) is available from Mobil Chemical Corp., Films Division, Pittsford, NY, under the designation 100 Dicor LBW. In both embodiments it is provided with a thickness of 0.001 inches (0.025 mm). The surfaces of the BOPP material are provided with treatments to increase its adhesion to materials such as ethylene acetate acrylic (EAA).

The EAA material is available from Dow Chemical, Midland, Michigan, under the designation Primacor 3440 and is tinted with 5% (by weight) white TiO₂ available from Spectrum Color, Minneapolis, MN. The EAA is coated to a thickness of 0.001 inches (0.025 mm).

The aluminum foil is available from Allfoils, Inc., Cleveland, OH under the designation type 1145, "A" wettability, with a thickness of 0.35 mil (0.00889 millimeter).

The inner heat sealable layer is preferably SURLYN brand resin, available from DuPont as described above. It is preferably modified with an anti-block compound, also available from E.I. DuPont, under the designation Conpol 5B10S1.

It will, of course, be understood that many other materials could be used to form the laminate sheet materials used in packages according to the present invention and that the preferred materials described above should not be construed as limiting the scope of the invention.

The laminate sheet materials used in packages constructed according to the present invention are manufactured in processes similar to those used to manufacture known laminate sheet materials incorporating metal foil vapor barriers where no fibers are present. Such manufacturing techniques are well known to those skilled in the art of producing packaging laminates and will not be described in detail herein.

Figure 4 does, however, illustrate a schematic diagram of one method of manufacturing a laminate sheet material 50 according to the present invention. As shown there, the method of manufacturing the embodiment of laminate sheet material 30 (see Fig. 3) is depicted.

To begin, a sheet of biaxially oriented polypropylene (BOPP) is extrusion mounted using a layer of EAA to the preferred barrier material, i.e., aluminum foil in the preferred embodiment. That composite laminate 46 (consisting of BOPP/EAA/foil) is then directed over nip roll 44 which runs between a chilled textured roll 42, both of which rotate as depicted. The foil side is exposed as laminate 46 runs over roll 44. An extrusion coater 40 deposits a layer of the preferred heat seal material (SURLYN resin # 1702) into the nip formed between rolls 42 and 44. Also directed over nip roll 44 are the fibers 45 according to the present invention.

The process depicted in Fig. 4 results in fibers 45 being contained within the heat seal layer of the laminate sheet material 50 produced by this method.

Alternately, it will be understood that fibers 25 depicted in Fig. 2 could be similarly mounted in a layer of EAA which is extrusion coated onto the vapor barrier 23 depicted in Fig. 2. In one preferred process, the layer 21 of biaxially oriented polypropylene (in the preferred embodiment) is extrusion coated over the layer of EAA 22 and fibers 25. The heat seal layer 24 of SURLYN resin or a similar material can be extrusion coated onto the vapor barrier layer either before or after the EAA and BOPP layers have been bonded to the opposing side of the vapor barrier layer 23.

Furthermore, although multi-layer laminate sheet materials with four or more layers are discussed above, it will also be understood that the use of fibers within two or three layer laminate sheet materials are contemplated. In that embodiment, the fiber(s) would be laminated between an inner and outer layer of material. Such laminates (without fibers) are well known and will not be described further herein. They are commonly used in packaging materials such as foods and other products which do not require the high level of hermeticity needed when packaging curable casting/splinting materials.

## Claims

1. A kit of orthopedic casting/splinting materials comprising:
a) a sheet (11) of curable casting/splinting material; and
b) a substantially hermetic package (10) containing the sheet (11) of curable casting/splinting material, the package (10) comprising:
1) laminate sheet material (20; 30) having opposite side edges, and an outer layer (21, 22; 31, 32) and a heat sealable inner layer (24; 34) bonded to the outer layer (21, 22; 31, 32); and
2) a heat seal (12, 14 and 16) bonding at least a portion of the inner layer (24; 34) of the laminate sheet material (20; 30) to form the package (10), the heat seal (12, 14 and 16) bonding the laminate sheet material (20; 30) to form opposite ends of the package (10), the ends of the package (10) defining a first direction extending between the ends and a second direction extending generally perpendicularly with respect to the first direction;
the kit being **characterized in that** a plurality of generally parallel fibers (25; 35) are substantially evenly spaced apart and disposed within the inner layer (34) or between the inner layer (24) and the outer layer (21, 22; 31, 32), the fibers extending substantially along the first direction to guide tearing the package (10) along the first direction, the fibers (25; 35) being substantially evenly spaced apart along the laminate sheet material (20; 30) across substantially the entire laminate sheet material (20; 30) in the second direction such that the distance between either side of the laminate sheet material (20; 30) and at least one fiber (25; 35) is no greater than the distance the fibers (25; 35) are spaced apart.

2. A kit according to claim 1 further
**characterized in that** the curable casting/splinting material (11) is moisture curable, and contains an isocyanate-functional resin.

3. A kit according to any of claims 1-2 further
**characterized in that** a portion of each fiber (25; 35) is contained within the heat seal (12, 14 and/or 16).

4. A kit according to any of claims 1-3 further
**characterized in that** the laminate sheet material (20; 30) further comprises an interior layer (23; 33) laminated between the inner layer (24; 34) and the outer layer (21, 22; 31, 32), the interior layer (23; 33) comprising a metal foil highly impervious to air and water vapor, the inner layer (24; 34) comprising a heat sealable plastic resin film (24; 34) laminated to the interior layer.

5. A kit according to claim 4 further
**characterized in that** the plurality of fibers (35) are disposed between the interior layer (33) and the inner layer (34).

6. A kit according to claim 4 further
**characterized in that** the plurality of fibers (25) are disposed between the interior layer (23) and the outer layer (21, 22).

7. A kit according to any of claims 1-6 further
**characterized in that** the fibers (25; 35) are substantially evenly spaced on approximately 25mm centers across the width of the package.

8. A kit according to any of claims 1-7 further
**characterized in that** the fibers (25; 35) each comprises a glass filament.

9. A kit according to claim 8 further
**characterized in that** the fibers (25; 35) each comprises a glass multi-filament fiber.

10. A kit according to any of claims 1-9 further
**characterized in that** each of the fibers (25; 35) has a nominal diameter of approximately 0.008 millimeters.

## Patentansprüche

1. Kit mit Materialien für orthopädische Steifverbände/Schienen, umfassend:
a) eine Bahn (11) aus härtbarem Material für Steifverbände/Schienen; und
b) eine im wesentlichen luftdichte Verpackung (10), die die Bahn (11) aus härtbarem Material für Steifverbände/Schienen enthält, wobei die Verpackung (10) folgendes umfasst:
1) Laminatfolienmaterial (20; 30) mit einander gegenüberliegenden Seitenkanten und einer äußeren Schicht (21, 22; 31, 32) und einer heißsiegelbaren inneren Schicht (24; 34), die mit der äußeren Schicht (21, 22; 31, 32) verklebt ist; und
2) eine Heißversiegelung (12, 14 und 16), die wenigstens einen Teil der inneren Schicht (24; 34) des Laminatfolienmaterials (20; 30) unter Bildung der Verpackung (10) verklebt, wobei die Heißversiegelung (12, 14 und 16) das Laminatfolienmaterial (20; 30) so verklebt, dass einander gegenüberliegende Enden der Verpackung (10) gebildet werden, wobei die Enden der Verpackung (10) eine erste Richtung, die sich zwischen den Enden erstreckt, und eine zweite Richtung, die sich im allgemeinen senkrecht zur ersten Richtung erstreckt, definieren;
wobei der Kit **dadurch gekennzeichnet** ist, dass eine Vielzahl von im allgemeinen parallelen Fasern (25; 35) sich im wesentlichen in gleichmäßigem Abstand voneinander innerhalb der inneren Schicht (34) oder zwischen der inneren Schicht (24) und der äußeren Schicht (21, 22; 31, 32) befinden, wobei sich die Fasern im wesentlichen entlang der ersten Richtung erstrecken, so dass sie das Aufreißen der Verpackung (10) entlang der ersten Richtung führen, wobei sich die Fasern (25; 35) über im wesentlichen das gesamte Laminatfolienmaterial (20; 30) hinweg in der zweiten Richtung im wesentlichen in gleichmäßigem Abstand voneinander entlang des Laminatfolienmaterials (20; 30) befinden, so dass der Abstand zwischen jeweils den beiden Seiten des Laminatfolienmaterials (20; 30) und wenigstens einer Faser (25; 35) nicht größer ist als der Abstand zwischen den Fasern (25; 35).

2. Kit gemäß Anspruch 1, weiterhin **dadurch gekennzeichnet**, dass das härtbare Material (11) für Steifverbände/Schienen feuchtigkeitshärtbar ist und ein isocyanatfunktionelles Harz enthält.

3. Kit gemäß einem der Ansprüche 1-2, weiterhin **dadurch gekennzeichnet**, dass ein Teil jeder Faser (25; 35) in der Heißversiegelung (12, 14 und/oder 16) enthalten ist.

4. Kit gemäß einem der Ansprüche 1-3, weiterhin **dadurch gekennzeichnet**, dass das Laminatfolienmaterial (20; 30) weiterhin eine Innenschicht (23; 33) umfasst, die zwischen die innere Schicht (24; 34) und die äußere Schicht (21, 22; 31, 32) laminiert ist, wobei die Innenschicht (23; 33) eine Metallfolie umfasst, die in hohem Maße undurchlässig für Luft und Wasserdampf ist, wobei die innere Schicht (24; 34) eine heißsiegelbare Kunstharzfolie (24; 34) umfasst, die auf die Innenschicht laminiert ist.

5. Kit gemäß Anspruch 4, weiterhin **dadurch gekennzeichnet**, dass sich die Vielzahl von Fasern (35) zwischen der Innenschicht (33) und der inneren Schicht (34) befindet.

6. Kit gemäß Anspruch 4, weiterhin **dadurch gekennzeichnet**, dass sich die Vielzahl von Fasern (35) zwischen der Innenschicht (23) und der äußeren Schicht (21, 22) befindet.

7. Kit gemäß einem der Ansprüche 1-6, weiterhin **dadurch gekennzeichnet**, dass sich die Fasern (25; 35) im wesentlichen in gleichmäßigem Abstand voneinander auf ungefähr 25 mm großen Zentren über die Breite der Verpackung befinden.

8. Kit gemäß einem der Ansprüche 1-7, weiterhin **dadurch gekennzeichnet**, dass die Fasern (25; 35) jeweils ein Glasfilament umfassen.

9. Kit gemäß Anspruch 8, weiterhin **dadurch gekennzeichnet**, dass die Fasern (25; 35) jeweils eine Glasmultifilamentfaser umfassen.

10. Kit gemäß einem der Ansprüche 1-9, weiterhin **dadurch gekennzeichnet**, dass jede der Fasern (25; 35) einen nominellen Durchmesser von ungefähr 0,008 Millimeter hat.

## Revendications

1. Kit de matériaux de pose d'attelle/moulage orthopédiques comprenant :
a) une feuille (11) d'un matériau de pose d'attelle/moulage durcissable ; et
b) un emballage essentiellement hermétique (10) contenant la feuille (11) d'un matériau de pose d'attelle/moulage durcissable, l'emballage (10) comprenant :
1) un matériau en feuilles stratifiées (20, 30) comportant des bords latéraux opposés et une couche externe (21, 22 ; 31, 32) et une couche interne thermoscellable (24 ; 34) collée à la couche externe (21, 22 ; 31, 32) ; et
2) un thermoscellage (12, 14 et 16) collant au moins une partie de la couche interne (24 ; 34) du matériau en feuilles stratifiées (20 ; 30) pour former l'emballage (10), le thermoscellage (12, 14 et 16) collant le matériau en feuilles stratifiées (20 ; 30) pour former les bords opposés de l'emballage (10), les extrémités de l'emballage (10) définissant une première direction s'étendant entre les extrémités et une seconde direction s'étendant de manière généralement perpendiculaire par rapport à la première direction ;
le kit étant caractérisé par le fait qu'une pluralité de fibres généralement parallèles (25 ; 35) sont sensiblement séparées de manière égale et disposées à l'intérieur de la couche interne (34) ou entre la couche interne (24) et la couche externe (21, 22 ; 31, 32), les fibres s'étendant essentiellement suivant la première direction pour guider le déchirage de l'emballage (10) suivant la première direction, les couches (25 ; 35) étant séparées de manière sensiblement égales suivant le matériau en feuilles stratifiées (20 ; 30) sensiblement à travers le matériau en feuilles stratifiées (20 ; 30) entier dans la seconde direction de manière telle que la distance séparant l'une ou l'autre face du matériau en feuilles stratifiées (20 ; 30) d'au moins une fibre (25 ; 35) ne soit pas plus grande que la distance par laquelle les fibres (25 ; 35) sont séparées entre elles.

2. Kit selon la revendication 1, caractérisé en outre en ce que le matériau de pose d'attelle/moulage durcissable (11) est durcissable à l'humidité, et contient une résine fonctionnelle isocyanate.

3. Kit selon l'une quelconque des revendications 1 et 2, caractérisé en outre en ce qu'une partie de chaque fibre (25 ; 35) est contenue dans le thermoscellage (12, 14 et/ou 16).

4. Kit selon l'une quelconque des revendications 1 à 3, caractérisé en outre en ce que le matériau en feuilles stratifiées (20 ; 30) comprend en outre une couche intérieure (23 ; 33) stratifiée entre la couche interne (24 ; 34) et la couche externe (21, 22 ; 31, 32), la couche intérieure (23 ; 33) comprenant une feuille métallique hautement imperméable à l'air et à la vapeur d'eau, la couche interne (24 ; 34) comprenant un film résineux en matière plastique thermoscellable (24 ; 34) stratifié sur la couche interne.

5. Kit selon la revendication 4, caractérisé en outre en ce que la pluralité de fibres (35) sont disposées entre la couche intérieure (33) et la couche interne (34).

6. Kit selon la revendication 4, caractérisé en outre en ce que la pluralité de fibres (25) sont disposées entre la couche intérieure (23) et la couche externe (21, 22).

7. Kit selon l'une quelconque des revendications 1 à 6, caractérisé en outre en ce que les fibres (25 ; 35) sont espacées de manière essentiellement régulières sur des centres d'approximativement 25 millimètres sur toute la largeur de l'emballage.

8. Kit selon l'une quelconque des revendications 1 à 7, caractérisé en outre en ce que les fibres (25 ; 35) comprennent chacune un filament de verre.

9. Kit selon la revendication 8, caractérisé en outre en ce que les fibres (25 ; 35) comprennent chacune une fibre multifilament de verre.

10. Kit selon l'une quelconque des revendications 1 à 9, caractérisé en outre en ce que chacune des fibres (25 ; 35) a un diamètre nominal d'approximativement 0,008 millimètre.
